# EUROPEAN PATENT SPECIFICATION

(11) **EP 0 289 138 B1**
(45) Date of publication and mention of the grant of the patent: **21.07.1993**
(21) Application number: 88302736.9
(22) Date of filing: 28.03.1988
(51) Int. Cl.: C12N 9/28, C12P 21/02, C12P 19/14

(54) **Maltotetraose-forming amylase**
Maltotetraose-bildende Amylase
Amylase formant du maltotétraose

(30) Priority: 28.03.1987 JP 74878/87
(43) Date of publication of application: 02.11.1988
(73) Proprietor: KABUSHIKI KAISHA HAYASHIBARA SEIBUTSU KAGAKU KENKYUJO, Okayama-shi Okayama (JP)
(72) Inventor: Kubota, Michio, Okayama-shi Okayama (JP); Nakada, Tetsuya, Okayama-shi Okayama (JP); Sakai, Shuzo, Akaiwa-gun Okayama (JP)
(74) Representative: Pendlebury, Anthony

(56) References cited:
- EP-A- 0 257 535
- EP-A- 0 298 645
- US-A- 3 654 082
- PATENT ABSTRACTS OF JAPAN, vol. 11, no. 30 (C-400)[2477], 29th January 1987
- PATENT ABSTRACTS OF JAPAN, vol. 11, no. 361 (C-459)[2308], 25th November 1987
- APPLIED MICROBIOLOGY BIOTECHNOLOGY, vol. 23, 1986, pages 355-360, Springer Verlag; R. NAKAJIMA et al.: "Comparison of amino acid sequences of eleven different alpha-amylases"
- Agric. Biol. Chem. (1983),47,1761-68

## Description

The present invention relates to a novel maltotetraose-forming amylase, and its preparation and uses.

### Definitions and abbreviations

Amino acid residues are abbreviated as follows:
- Ala:: alanine residue
- Arg:: arginine residue
- Asn:: asparagine residue
- Asp:: aspartic acid residue
- Gln:: glutamine residue
- Glu:: glutamic acid residue
- Gly:: glycine residue
- His:: histidine residue
- Ile:: isoleucine residue
- Leu:: leucine residue
- Lys:: lysine residue
- Pro:: proline residue
- Ser:: serine residue
- Tyr:: tyrosine residue
- Val:: valine residue
The terms "high maltotetraose" and "high maltotetraitol" designate a saccharified starch product having a relatively high maltotetraose content, and its hydrogenated derivative respectively.

"d.s.b." and "DE" are abbreviations for dry solids basis and dextrose equivalent.

Glucose and maltose are generally prepared on an industrial-scale by saccharifying starch with amylases. There has been considerable interest in amylases that can be used in the preparation of maltooligosaccharides.

In particular, maltotetraose has drawn attention as a partial starch hydrolysate having a moderate sweetness and a low saccharide content, and establishment of its industrial-scale preparation has been in great demand.

As disclosed, for example, in United States Patent No. 3,654,082; John F. Robyt and Rosalie J. Ackerman, Archives of Biochemistry and Biophysics, Vol. 145, pp. 105-114 (1971); Jurgen Schmidt and Michael John, Biochimica et Biophysica Acta, Vol. 566, pp. 88-99 (1979); Yoshiyuki Sakano et al., Journal of the Japanese Society of Starch Science, Vol. 29, No. 2, pp. 131-137 (1982); Yoshiyuki Sakano et al., Agricultural and Biological Chemistry, Vol. 46, No. 3, pp. 639-646 (1982); and Yoshiyuki Sakano et al., ibid., Vol. 47, No. 8, pp. 1761-1768 (1983), it has been known for years that Pseudomonas stutzeri NRRL B-3389 produces a maltotetraose-forming enzyme or maltotetraose-forming amylase (EC 3.2.1.60).

The maltotetraose-forming amylase derived from Pseudomonas stutzeri NRRL B-3389, as reported in the above publications, is relatively low in optimum-temperature and stable-temperature, and its thermostability is insufficient in industrial uses.

Shyoich Kobayashi discloses in Japanese Patent Laid-Open No. 202,687/86, page 2, right upper corner, line 1-4, that this amylase is produced by a bacterium of the species Pseudomonas stutzeri and is low in producibility (2-5 IU/ml); thus this amylase is not suitable for industrial uses.

We have unexpectedly found that a bacterium of the species Pseudomonas stutzeri produces a novel maltotetraose-forming amylase having an improved thermostability.

Accordingly the present invention provides a malto-tetraose-forming amylase having the following physico-chemical properties:
(a) Action
   Acting on starch to produce primarily maltotetraose
b) Substrate specificity
   Acting on starch, amylose, amylopectin, glycogen and β-limit dextrin; but substantially not acting on cyclodextrin, dextran, pullulan and elsinan
(c) Optimum pH
   About pH 7.0-7.5 at 40°C for 20 minutes
(d) Stable pH
   About pH 6.5-9.5 at 40°C for 1 hour
(e) Optimum temperature
   About 50°C at pH 7.0 for 20 minutes
(f) Stable temperature
   Up to about 45°C at pH 7.0 for 1 hour. The activity is substantially retained at about 50°C in the presence of Ca²⁺, while at about 55°C about 70% of the activity is retained
(g) Inhibition and stabilization
   Inhibited by Hg⁺
   Thermally stabilized by Ca²⁺
(h) Molecular weight
   46,000±1,000 on SDS-polyacrylamide gel electrophoresis
   47,000±2,000 on ultracentrifugal analysis
(i) Isoelectric point (pI)
   About pI 4.8 on polyacrylamide gel isoelectric electrophoresis
(j) Ultraviolet absorption
   About 275-280 nm.

Some typical properties of the maltotetraose-forming amylase are compared with those of the maltotetraose-forming amylase derived from Pseudomonas stutzeri NRRL B-3389 reported in Agricultural and Biological Chemistry, Vol. 47, No. 8, pp. 1761-1768 (1983). The results are as follows:
(1) The optimum pH of the present amylase is wide (i.e. about 7.0-7.5), while that of the amylase derived from Pseudomonas stutzeri NRRL B-3389 is about 8.0.
(2) The stable pH range of the present amylase is wide (i.e. about 6.5-9.5), while that of the amylase derived from Pseudomonas stutzeri NRRL B-3389 is about 6.5-8.5.
(3) The optimum temperature of the present amylase is relatively high (i.e. about 50°C), while that of the amylase derived from Pseudomonas stutzeri NRRL B-3389 is about 45°C.
(4) The stable-temperature of the present amylase is about 45°C (about 50°C in the presence of Ca⁺), while that of the amylase derived from Pseudomonas stutzeri NRRL B-3389 is about 40°C.
(5) The molecular weight of the present amylase is 46,000±1,000 on polyacrylamide gel electrophoresis, while that of the amylase derived from Pseudomonas stutzeri NRRL B-3389 is 55,000.
(6) The pI of the present amylase is about 4.8, while that of the amylase derived from Pseudomonas stutzeri NRRL B-3389 is 5.6 or 5.3.

As described above, the present maltotetraose-forming amylase is superior in pH stability and thermostability than the amylase derived from Pseudomonas stutzeri NRRL B-3389. These facts confirm that the present maltotetraose-forming amylase is novel and is suitable for use in industrial applications.

Any bacterium can be used in the present invention as long as it produces the maltotetraose-forming amylase.

Pseudomonas stutzeri MO-19, as described hereinafter, or a mutant thereof can be favorably used in the invention.

Pseudomonas stutzeri MO-19 was found and isolated from a soil sample in Kayo-cho, Jobo-gun, Okayama, Japan. A deposit of the bacterium was made on February 13, 1987 at the Fermentation Research Institute, Agency of Industrial Science and Technology, Ministry of International Trade and Industry, 1-3, Higashi, 1-chome, Tsukuba-shi, Ibaraki-ken, 305, Japan, and has been allocated the Deposit Number FERM BP-1682.

The following is the taxomony of Pseudomonas stutzeri MO-19.
A. Cell morphology:
   (a) Cell form and size
      Rod-shaped, 0.5-0.6x1.0-1.2 micrometers.

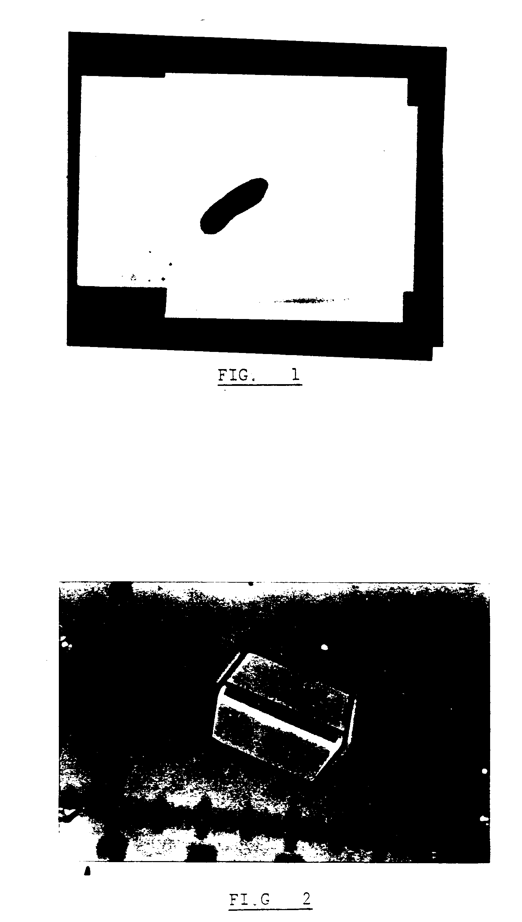

      FIG. 1 of the accompanying drawings shows an electron microscopic figure (x10,000) of the cells of Pseudomonas stutzeri MO-19 (FERM BP-1682).
   (b) Cell polymorphism
      Not present
   (c) Motility
      Motile
   (d) Flagellum conditions
      Polar monotrichous
   (e) Spore and sporangium
      Not forming
   (f) Gram reaction
      Negative
   (g) Acid-fast
      Negative
B. Cultivation properties:
   (a) Cultivation on meat broth agar plate (28°C, 2 days)
      Cell growth is relatively fast and its colony is 1-3 mm in diameter. The colony has an opaque, glistening and brownish yellow oval form, as well as having a smooth surface.
   (b) Cultivation on meat broth agar slant (28°C, 2 days)
      Cell growth is moderate. Colony has an opaque, glistening and brownish yellow feather-like form, as well as having a plane rise.
   (c) Cultivation in a meat broth liquid culture medium (28°C, 2 days)
      After one-day cultivation, the bacterium renders the culture medium slightly turbid, and, after 2-day cultivation, the culture medium becomes strongly turbid. Cell growth is fast, and gas or pigment is not formed.
   (d) Meat broth stab culture (28°C, 2 days)
      Growing in chain-like form around the stabbed area
   (e) Meat broth gelatin stab culture
      Not liquefying gelatin
   (f) Litmus and milk (28°C, 5 days)
      The culture medium becomes alkaline within 5-day cultivation, and no solidification of the milk is observed.
C. Physiological properties:
   (a) Reduction of nitrate
      Positive
   (b) Denitrification reaction
      Positive
   (c) M-R reaction
      Negative
   (d) V-R test
      Negative
   (e) Production of indol
      Negative
   (f) Production of H₂S
      Positive
   (g) Hydrolysis of starch
      Hydrolyzed
   (h) Utilization of citrates
      Positive
   (i) Utilization of inorganic nitrogen
      Ammonium salts and nitrates are both utilized
   (j) Production of pigment
      Not producing soluble pigment
   (k) Oxidase
      Positive
   (l) Catalase
      Positive
   (m) Temperature- and pH-ranges for cell growth
      Cell grows fast in a pH range of 4-7, but does not grow at pH 2 or lower, or pH 8 or higher. Cell grows fast at a temperature in the range of 16-35°C, but does not grow at a temperature of 2°C or lower, or 42°C or higher
   (n) Cell growth in the presence of oxygen
      Aerobic
   (o) O-F test
      Oxidative
   (p) Acid and gas formation from saccharide
      Acid is formed from D-glucose, D-mannose, lactose, maltose and starch, but not formed from L-arabinose, D-xylose, D-fructose, sucrose and glycerol. Gas is formed from D-mannose but not formed from the other saccharides
   (q) Growth pH
      pH 7.7 (proteose peptone glucose medium)
   (r) Hydrolysis of cellulose
      Negative

Based on the above described taxonomy and with reference to Bergey's Manual of Determinative Bacteriology, 7th edition (1957) and 8th edition (1974), the bacterium was identified as a bacterium of the species Pseudomonas stutzeri.

The present maltotetraose-forming amylase can be prepared with cultivation methods that are used in conventional bacterium cultivation such as liquid or solid culture. The liquid culture prefers shaking or agitation-aeration conditions to static conditions. Cultivation media containing a carbon source, a nitrogen source and minerals, for example, synthetic-, semisynthetic- and natural-media, can be favorably used as long as bacteria of the genus Pseudomonas grow therein.

More particularly, culture media containing an amylaceous substance, for example, starch, amylopectin and partial starch hydrolysate, together with a calcium salt can be favorably used in order to accelerate the formation of the maltotetraose-forming amylase.

Usually, bacteria of the genus Pseudomonas are inoculated onto a neutral or slightly alkaline culture medium, and then cultured at a temperature of about 20-35°C for about 0.5-5 days.

The culture thus obtained is usually used intact, or prepared into supernatant or filtrate by removing the cell debris from the culture, prior to its use.

If necessary, the maltotetraose-forming amylase can be purified with salting out using (NH₄)₂SO₄ or Na₂SO₄, precipitation using acetone or ethanol, and chromatography such as gel-, ion exchange- or affinity-chromatography, prior to its use. The purified maltotetraose-forming amylase can be immobilized with conventional methods including carrier-linkage, cross-linkage and entrapment.

In industrial preparation of high maltotetraose using the present maltotetraose-forming amylase, desirably, an amylaceous substance, for example, starch, amylopectin, amylose and partial starch hydrolysate, is used as the substrate.

In preparation of food products containing amylaceous substances, they can be subjected to the action of the maltotetraose-forming amylase to form maltotetraose therein in order to prevent possible retrogradation of the amylaceous substances, if necessary.

Usually, a solution containing about 5-45 w/v % amylaceous substance is added with the maltotetraose-forming amylase in an amount of amount 1-20 units/g amylaceous substance, and the enzymatic reaction is continued at pH 5-9 and about 40-70°C for 0.5-3 days.

In this case, the presence of about 0.0005-0.05 mole CaCl₂ in reaction solution improves the thermostability of the maltotetraose-forming amylase, as well as facilitating its enzymatic reaction.

In order to increase the maltotetraose content in a reaction mixture to the possible highest level, desirably, an amylaceous substance having the possible lowest degree of liquefaction by acid or α-amylase, in other words, DE of 15 or lower, preferably, lower than 6, is subjected to the action of the maltotetraose-forming amylase.

The composition and content of the obtained high maltotetraose can be freely changed and increased to meet its final use by using the maltotetraose-forming amylase in combination with other starch-degrading enzymes, for example, cyclomaltodextrin glucanotransferase (EC 2.4.1.19), α-amylase (EC 3.2.1.1), β-amylase (EC 3.2.1.2), glucoamylase (EC 3.2.1.3), α-glucosidase (EC 3.2.1.20), pullulanase (EC 3.2.1.41) and isoamylase (EC 3.2.1.68).

More particularly, the maltotetraose-forming amylase can be advantageously used together with one or more starch debranching enzymes, for example, pullulanase and isoamylase, in order to increase the content of a high maltotetraose to 60-80 w/w %, d.s.b.

The content of a high maltotetraose having about 40-80 w/w %, d.s.b., can be freely increased by removing contaminating saccharides and dextrins therefrom with a suitable method such as fractionation.

Examples of such fractionation include that using semipermeable membranes as disclosed in Japanese Patent Laid-Open No. 4647/73; that using organic precipitants as disclosed in Japanese Patent Laid-Open No. 102854/74, and that using strongly-acidic cation exchange resins as disclosed in Japanese Patent Laid-Open No. 148794/84, and a high maltotetraose having the possible highest purity not lower than 98 w/w % can be easily obtained by employing the fractionation, if necessary.

Thereafter, the obtained high maltotetraose is usually filtered, purified with decoloration using activated charcoal and deionization using ion exchange resins in H- and OH-form, and concentrated into syrup which can be freely dried into powder, if necessary.

The high maltotetraose thus obtained contains 40 w/w % or more maltotetraose, d.s.b.

High maltotetraose can be advantageously reduced into a chemically stabler high maltotetraitol.

For example, an about 40-60% aqueous solution of high maltotetraose is placed in an autoclave, and added with about 8-10% Raney Nickel as the catalyst. The mixture is heated to 90-140°C while stirring, and hydrogenated at this temperature under a hydrogen pressure of 20-150 kg/cm². After completion of the hydrogenation, the Raney Nickel is removed from the reaction mixture, and the residue is purified with activated charcoal and ion exchange resins similarly as in the preparation of high maltotetraose. The resultant is concentrated into syrup which may be dried into powder.

The obtained high maltotetraitol usually contains 40 w/w % or more maltotetraitol, d.s.b.

High maltotetraose and high maltotetraitol, obtained by the above described methods, can be extensively used in food products as a sweetener with a moderate sweetness, as well as a body-imparting agent, humectant, gloss-imparting agent, adhesive, flavor-retaining agent, viscosity-controlling agent, crystallization-preventing agent, sticking-preventing agent for candy, retrogradation-preventing agent, and nutrient supplement.

For example, coating of food products with a heated syrup of high maltotetraose or high maltotetraitol has the features that it hardens and dries faster the food products and causes less sticking than in the case of using conventional saccharides, as well as that it imparts excellent gloss and texture to the food products.

High maltotetraose and high maltotetraitol can be used alone, or, if necessary, in combination with one or more sweeteners, for example, glucose, maltose, isomerized sugar, honey, maple sugar, sorbitol, maltitol, paratinose, dihydrochalcone, stevioside, α-glycosyl stevioside, sweet substance derived from Momordica grosvenori Swingle, glycyrrhizin, α-glycosyl glycyrrhizin, L-asparatyl-L-phenylalanine methyl ester, saccharin, glycine and alanine.

Since high maltotetraose and high maltotetraitol well harmonize with substances effecting acidity-, salty-, astringent-, delicious- or bitter-taste and exert a high thermal resistance, they can be favorably used in food products in order to sweeten and/or season them, as well as to improve their taste quality.

High maltotetraose and high maltotetraitol can be freely used in seasonings, for example, soy sause, powdered soy sause, "miso (a kind of Japanese seasoning prepared by fermenting rice, wheat, barley and beans)", "funmatsu-miso (powdered miso)", "moromi (an unrefined sake)", "hishio (a salted meat)", "furikake (a seasoned fish meal)", mayonnaise, dressing, vinegar, "sanbai-zu (a mixture seasoning comprising soy sauce, vinegar, sake and sugar)", "funmatsu-sushi-no-moto (a premix for seasoning sushi)", "chuka-no-moto (an instant mix of Chinese dish)", "tentsuyu (a sause for Japanese deep-fat fried food)", "mentsuyu (a sause for Japanese vermicelli)", sause, catsup, "yakiniku-no-tare (a sause for Japanese grilled meat)", curry roux, instant stew mix, instant soup mix, "dashi-no-moto (an instant stock mix)", mixed seasoning, "mirin (a sweet sake)", "shin-mirin (a synthetic mirin)", table syrup and coffee sugar.

High maltotetraose and high maltotetraitol can be freely used to sweeten food products, for example, "wagashi (Japanese-style confectioneries)" such as "senbei (rice crackers)", "arare-mochi (pellet-shaped senbei)", "okoshi (a millet-and-rice cake)", rice paste, "manju (a bun with a bean-jam filling)", "uiro (a sweet rice jelly)", "an (a bean jam)", "yokan (a sweet jelly of beans)", "mizu-yokan (a soft adzuki-bean jelly)", "kingyoku (a kind of yokan)", jelly, "pao de Castella (a sponge cake)" and "amedama (toffees)"; confectioneries and bakery products such as bun, biscuit, cracker, cookie, pie, pudding, butter cream, custard cream, cream puff, waffle, sponge cake, doughnut, chocolate, chewing gum, caramel and candy; frozen desserts such as ice cream and shurbet; syrups such as "kajitsu-no-syrup-zuke (a preserved fruit)" and "kori-mitsu (a sugar syrup for shaved ice)"; processed grains such as noodles, rices and artificial meats; pastes such as flour paste, peanut paste, fruit paste and spread; processed fruits and vegetables such as jam, marmalade, "syrup-zuke (fruit pickles)", and "toka (conserves)", pickles and pickled products such as "fukujin-zuke (red colored radish pickles)", "bettara-zuke (a kind of whole fresh radish pickles)", "senmai-zuke (a kind of sliced fresh radish pickles)" and "rakkyo-zuke (pickled shallots)", premixes for pickles and pickled products such as "takuan-zukeno-moto (a premix for pickled radish)" and "hakusai-zuke-no-moto (a premix for fresh white rape pickles)"; meat products such as ham and sausage; fish meat products such as fish ham, fish sausage, "kamaboko (a steamed fish paste)", "chikuwa (literally bamboo wheels)", and "tenpura (a Japanese deep-fat fried fish paste)"; "chinmi (relish)" such as "uni-no-shiokara (salted guts of sea urchin)", "ikura-no-shiokara (salted guts of squid)", "su-konbu (a processed tangle)", "saki-surume (dried squid strips)" and "fugu-no-mirinboshi (a dried mirin-seasoned swellfish)"; "tsukudani (foods boiled down in soy)", such as those of laver, edible wild plants, dried squid, fish and shellfish; daily dishes such as "nimane (cooked beans)", potato salad and "konbu-maki (a tangled roll)"; milk products such as processed cheese and milk powder; canned and bottled products such as those of meat, fish meat, fruit and vegetable; alcoholic beverages such as synthetic sake, fruit wine and liquors; soft drinks such as coffee, cocoa, juice, carbonated beverage, sour milk beverage and beverage containing a lactic bacterium; and instant food products such as instant pudding mix, instant hot cake mix, juice powder, instant coffee "sokuseki-shiruko (an instant mix of adzuki-bean soup with rice cake)" and instant soup mix, as well as to improve their taste quality and physical properties.

High maltotetraose and high maltotetraitol can be advantageously used in nutrient supplements, for example, those for hemodialysis, fluid foods for intubation feeding or baby foods for sick persons, convalescent feeble persons, the aged or infants who can not take normal food products.

In this case, one can prepare a liquid nutrient supplement, which can be used without further processings, as well as preparing a solid nutrient supplement, for example, those in powder and granules, which is dissolved, for example, in water, salt solution, fruit juice and milk, prior to its use.

High maltotetraose and high maltotetraitol can be used in feeds and pet foods for domestic animal and fowl, honey bee, silkworm and fish in order to improve their taste quality.

In addition, high maltotetraose and high maltotetraitol can be used to sweeten tobaaccos, cosmetics, toiletries and pharmaceuticals in solid, paste of liquid form, such as cigar, cigarette, dentrifrice, lipstic, lipcream medicine for internal administration, trouch, liver oil drop, oral refreshment agent, cachou and collutrium, as well as to improve their taste quality.

As described above, the working "food products" as referred to in the present invention shall mean all products that can be orally taken to taste them, as well as meaning nutrient supplements such as fluid food for intubation feeding.

To incorporate high maltotetraose and/or high maltotetraitol into food products, and conventional method can be employed so far as high maltotetraose and/or high maltotetraitol can be incorporated into food products, before completion of their processings therewith, for example, kneading, mixing, dissolving, melting, impregnating, applying, coating, spraying and injecting.

The following Examples further illustrate the present invention.

### Assay of the maltotetraose-forming amylase

Five milliliters of 1.0 w/v % soluble starch (pH 7.0) is added with 0.2 ml of an enzyme solution, and the mixture is incubated at 40°C for 20 minutes to effect enzymatic reaction. Thereafter, the reaction mixture is added with the Somogyi's reagent to suspend the reaction. The resultant reducing sugar is measured by the Nelson-Somogyi method. One unit of the malto-tetraose-forming amylase is defined as the amount of the enzyme that splits 1 micromole of glucosidic bonds per minute.

### Example 1

### Maltotetraose-forming amylase

A liquid culture medium containing 3 w/v % of soluble starch, 0.5 w/v % of corn steep liquor, 0.2 w/v % of polypeptone, 0.2 w/v % of K₂HPO₄, 0.05 w/w % of CaCl₂·2H₂O and water was adjusted to pH 7.2. One hundred milliliter aliquots of the liquid culture medium were placed in 500 ml-shake-flasks, and autoclaved at 120°C for 20 minutes. A seed culture of Pseudomonas stutzeri MO-19 was inoculated to each aliquot of the culture medium, and cultured at 27°C for 3 days under stirring conditions.

The maltotetraose-forming amylase activity in the resultant culture was about 250 units/ml.

A supernatant containing amylase obtained by centrifugal separation of the culture can be advantageously used to prepare the high maltotetraose from amylaceous substances.

### Example 2

### Maltotetraose-forming amylase

Fifteen liters of a liquid culture medium containing 4 w/v % of liquefied starch, 1.0 w/v % of corn steep liquor, 0.5 w/v % of polypeptone, 0.2 w/v % of NH₄NO₃, 0.2 w/v % of K₂HPO₄, 0.05 w/v % of CaCl₂·2H₂O and water was placed in a 30-liter jar fermentor, adjusted to pH 7.0, and autoclaved at 120°C for 20 minutes. One v/v % of a seed culture of Pseudomonas stutzeri MO-19 was inoculated to the liquid culture medium, and cultured at 28°C for 4 days under agitation-aeration conditions. The activity of maltotetraose-forming amylase in the resultant culture was about 220 units/ml. The culture was then filtered with a membrane filter, and the filtrate was salted out with (NH₄)₂SO₄ to collect the fraction that precipitated at 0.2-0.4 saturation. The fraction was chromatographed on "DEAE-Toyopearl 650S"®, an anion exchange resin commercialized by Toyo Soda Mfg. Co., Ltd., Tokyo, Japan, and "Mono Q"®, an anion exchange resin commercialized by Pharmacia LKB Biotechnology Inc., Uppsala, Sweeden, to obtain a solution containing a high-purity maltotetraose-forming amylase which showed a single protein band on polyacrylamide gel electrophoresis.

This purification increased the specific activity of the maltotetraose-forming amylase by about 4.3-folds, and the yield was about 48%. The obtained maltotetraose-forming amylase with a specific activity of 680±60 units/mg protein had the following physicochemical properties:
(a) Action
   Acting on starch to mainly produce maltotetraose
(b) Substrate specificity
   Acting on starch, amylose, amylopectin, glycogen and β-limit dextrin; but substantially not acting on cyclodextrin, dextran, pullulan and elsinan
(c) Optimum pH
   About pH 7.0-7.5 at 40°C for 20 minutes
(d) Stable pH
   About pH 6.5-9.5 at 40°C for 1 hour
(e) Optimum temperature
   About 50°C at pH 7.0 for 20 minutes
(e) Optimum temperature
   About 50°C at pH 7.0 for 20 minutes
(f) Stable temperature
   Up to about 45°C at pH 7.0 for 1 hour. The activity is substantially retained at about 50°C in the presence of Ca²⁺, while at about 55°C about 70% of the activity is retained.
(g) Inhibition and stabilization
   Inhibited by Hg⁺
   Thermally stabilized by Ca²⁺
(h) Molecular weight
   46,000±1,000 on SDS-polyacrylamide gel electrophoresis
   47,000±2,000 on ultracentrifugal analysis (when calculated as (partial specific volume)×(density)=0.75)
(i) Isoelectric point (pI)
   About pI 4.8 on polyacrylamide gel isoelectric electrophoresis
(j) Ultraviolet absorption
   About 275-280 nm

One-month standing of about 1.0 w/v % high-purity maltotetraose-forming amylase in 10 mM of Tris-HCl buffer (pH 8.0) at 4°C attained crystals. FIG. 2 of the accompanying drawings shows one of the crystals magnified 220 times.

The high-purity maltotetraose-forming amylase was with high-performance liquid chromatography in accordance with the method described by Rodney M. Hewick et al., The Journal of Biological Chemistry, Vol. 256, No. 15, pp. 7990-7997 (1981).

As a result, the sequence of 20 N-terminal amino acid residues of the maltotetraose-forming amylase was Asp-Gln-Ala-Gly-Lys-Ser-Pro-Asn-Ala-Val-Arg-Tyr-His-Gly-Gly-Asp-Glu-Ile-Ile-Leu (each amino acid residue is in L-configuration).

Similarly as the maltotetraose-forming preparation in Example 1, the high-purity maltotetraose-forming amylase, obtained in this Example, can be favorably used to prepare high maltotetraose from amylaceous substances.

### Example 3

### High maltotetraose

One part by weight of corn starch was added with 3.0 parts by weight of water containing 0.1 w/w % of "Termamyl 60L"®, an α-amylase commercialized by Novo industry A/S, Copenhagen, Denmark, per starch. The mixture was adjusted to pH 6.5, and allowed to stand at 95-100°C to gelatinize and liquefy the corn starch. Immediately after the DE of the reaction mixture reached 4.5, the reaction mixture was heated at about 120°C for 5 minutes, and then quickly cooled to 60°C.

The reaction mixture was then added with 4 units/g starch of a supernatant containing maltotetraose-forming amylase obtained by the method in Example 1, and kept at pH 6.5 and 55°C for 46 hours to effect saccharification.

The resultant saccharified product was heated to inactivate the amylase, decolored with activated charcoal, and deionized with ion exchange resins in H- and OH-form to obtain a high maltotetraose syrup with a moisture content of 25 w/w % in the yield of 95%, d.s.b., against the material starch.

The product containing about 55% maltotetraose, d.s.b., had about 25% sweetening power of sucrose.

The product can be extensively used in food products as a sweetener with a moderate sweetness, a low saccharide content and an excellent assimilability, as well as a body-imparting agent, viscosity-controlling agent, humectant, gloss-imparting agent, adhesive, flavor-retaining agent, crystallization-preventing agent, and sticking-preventing agent for candy.

### Example 4

### High maltotetraose

Six parts by weight of water containing 0.01 w/w % "Neo-Spitase"®, an α-amylase commercialized by Nagase Biochemicals, Ltd., Kyoto, Japan, per starch was mixed with 1 part by weight of potato starch while stirring. The mixture was adjusted to pH 6.0, and heated at 85-90°C to gelatinize and liquefy the starch. The resultant mixture was immediately heated to 120°C within 5 minutes in order to retain its DE to a level lower than 1.0, quickly cooled to 55°C, and adjusted to pH 7.0. To the reaction mixture was added "pullulanase (EC 3.2.1.41)", a product of Hayashibara Biochemical Laboratories Inc., Okayama, Japan, and a high-maltotetraose-forming amylase solution, obtained by the method in Example 2, in respective amount of 150 units/g starch and 8 units/g starch, and the resultant was kept at pH 7.0 and 50°C for 36 hours to effect saccharification.

The product was purified and concentrated similarly as in Example 3, and then spray-dried to obtain a powder with a moisture content lower than 1 w/w %.

The yield was about 93%, d.s.b., against the material starch.

The product containing about 76% maltotetraose, d.s.b., had 25% sweetening power of sucrose.

Similarly as the product in Example 3, this product can be extensively used in food products as a sweetener with a moderate sweetness, a low saccharide content and an excellent assimilability.

### Example 5

### High maltotetraose

The maltotetraose content of a product obtained by the method in Example 4 was increased by using a strongly-acidic cation exchange resin.

A product obtained by the method in Example 4 was dissolved to give a concentration of 60 w/w %, and then used as feed solution. "XT-1007® (in alkaline metal form)", a strongly-acidic cation exchange resin commercialized by Tokyo Chemical Industries, Kita-ku, Tokyo, Japan, was packed in 4 jacketted stainless steel columns, 5.4 cm in diameter, and the columns were cascaded to give a total bed depth of 20 m.

The feed solution was admitted to the columns in an amount of 5 v/v % against the resin, and 55°C water was passed through the columns at a space velocity (SV) of 0.13 to effect fractionation, followed by recovery of the fractions containing 90% or more maltotetraose. The fractions were purified and concentrated similarly as in Example 3 to obtain a syrup with a moisture content of 25 w/w % in the yield of about 60%, d.s.b., against the material saccharide. The product containing about 93% maltotetraose, d.s.b., had about 25% sweetening power of sucrose.

Similarly as the product in Example 3, the product can be extensively used in food products as a sweetener with a moderate sweetness, a low saccharide content and an excellent assimilability.

### Example 6

### High maltotetraitol

A product obtained by the method in Example 3 was prepared into 50% aqueous solution. The aqueous solution was placed in an autoclave, added with 10% of Raney Nickel as the catalyst, and heated to 90-125°C under a hydrogen pressure of 20-100 kg/cm² to complete hydrogenation while stirring.

The Raney Nickel was removed from the reaction mixture, and the residue was purified and concentrated similarly as in Example 3 to obtain a high maltotetraitol syrup with a moisture content of 25 w/w % in the yield of about 92%, d.s.b., against the material high maltotetraose.

The product containing about 55% maltotetraitol, d.s.b., had about 30% sweetening power of sucrose.

The product can be extensively used in food products as a sweetener with a moderate sweetness but without reducing ability, as well as a body-imparting agent, viscosity-controlling agent, humectant, gloss-imparting agent, adhesive, flavor-retaining agent, crystallization-preventing agent and sticking-preventing agent for candy.

### Example 7

### High maltotetraitol

A product obtained by the method in Example 4 was hydrogenated, purified and concentrated by the method in Example 6 to obtain a high maltotetraitol with a moisture content of about 25 w/w % in the yield of about 92%, d.s.b., against the material high maltotetraose.

The product containing about 76% maltotetraitol, d.s.b., had about 30% sweetening power of sucrose.

Similarly as the product in Example 6, the product can be extensively used in food products as a sweetener with a moderate sweetness and excellent assimilability but without reducing ability.

### Example 8

### Sweetener

One part by weight of a syrup obtained by the method in Example 5 was mixed homogeneously with 0.02 parts by weight of "α-G-Sweet"®, α-glycosyl stevioside syrup commercialized by Toyo Sugar Refining Co., Ltd., Tokyo, Japan, to obtain a syrup sweetener. The syrup sweetener with an excellent sweetness has an about 2-fold stronger sweetening power of sucrose, and its calorie is about one-half of sucrose with respect to the sweetening power.

Thus, the syrup sweetener can be favorably used to sweeten low-caloric food products for those whose calorie intakes are restricted such as obeses and diabetics.

The syrup sweetener can be favorably used to sweeten dental caries-preventing food products because it induces less formation of acid and insoluble glucan by dental caries-inducing microorganism.

### Example 9

### Custard Cream

Five hundred parts by weight of corn starch, 400 parts by weight of a powder obtained by the method in Example 4, 500 parts by weight of maltose, and 5 parts by weight of table salt were homogeneously mixed through mesh. The mixture was added with 1,400 parts by weight of egg, stirred and added with 5,000 parts by weight of boiling milk.

Thereafter, the resultant was stirred while heating with a slow fire, and the heating was stopped when the corn starch was completely gelatinized and become entirely semi-permeable. The product was cooled and added with a small amount of vanilla flavor to obtain a custard cream.

The product has a smooth texture, excellent gloss and moderate sweetness.

### Example 10

### "Uiro (a sweet rice jelly)"

Ninety parts by weight of rice powder was kneaded with 20 parts by weight of corn starch, 20 parts by weight of sugar, 1 part by weight of "maccha ( a powdered green tea)", 90 parts by weight of a syrup obtained by the method in Example 7, and an appropriate amount of water. The mixture was placed in a vessel, and steamed for 60 minutes to obtain "uiro".

The product was excellent in gloss, biting properties, flavor and taste. The product was stable over a long period of time because retrogradation of its amylaceous constituent was suppressed.

### Example 11

### Hard candy

Ninety parts by weight of sucrose was dissolved in 70 parts by weight of a syrup obtained by the method in Example 5, and the mixture was concentrated in vacuo to give a moisture content below about 2 w/w % while heating. The resultant was mixed with 0.15 parts by weight of citric acid and small amounts of flavors including lemon flavor. The resultant was then shaped in conventional manner to obtain a hard candy.

The hard candy is appropriately crisp because it effects less moisture adsorption and scarcely causes sticking.

### Example 12

### "Tsukudani (foods boiled down in soy)"

Two hundred and fifty parts by weight of tangle which had been treated to remove the sand sticked thereto, soaked in acid solution and cut into squares in conventional manner was added with 212 parts by weight of soy source, 300 parts by weight of amino acid solution, 40 parts by weight of sugar, and 20 parts by weight of a syrup obtained by the method in Example 3. Then, the mixture was added with 12 parts by weight of sodium glutamic acid, 8 parts by weight of caramel and 21 parts by weight of "mirin" while boiling, and the resultant was boiled down to obtain "konbu-no-tsukudani (a tsukudani of tangle)".

The product is an appetizing "konbu-no-tsukudani" with an excellent color, gloss, taste and flavor.

### Example 13

### "Bettara-zuke (a kind of whole fresh radish pickles)"

Four parts by weight of a syrup sweetener obtained by the method in Example 8, 0.05 parts by weight of licorice preparation, 0.008 parts by weight of malic acid, 0.07 parts by weight of sodium glutamate, 0.03 parts by weight of potassium sorbinate, and 0.2 parts by weight of pullulan were mixed homogeneously to obtain "bettara-zuke-no-moto (a premix for bettara-zuke)".

Thirty kilograms of Japanese radish was pickled with following salt, sugar and a solution prepared by using 4 kg of "bettarzuke-no-moto" to obtain "bettara-zuke".

The product was moderately sweet and excellent in biting properties, texture, gloss and flavor.

### Example 14

### Fluid food for intubation feeding

A composition was prepared with following: 550 parts by weight of a high-maltotetraose powder obtained by the method in Example 4; 50 parts by weight of "FINETOSE"®, an anhydrous crystalline α-maltose commercialized by Hayashibara Co., Ltd., Okayama, Japan; 190 parts by weight of dried egg-yolk; 209 parts by weight of milk powder; 4.4 parts by weight of NaCl; 1.85 parts by weight of KCl; 4 parts by weight of MgSO₄; 0.01 part by weight of thiamine; 0.1 part by weight of ascorbic acid; 0.6 parts by weight of vitamin E acetate; and 0.04 parts by weight of nicotinamide.

Twenty-five gram aliquots of the composition were packaged in laminated aluminum bags, and the bags were heat-sealed to obtain a solid nutrient supplement.

The product excellent in free-flowing ability, solubility and dispersibility, requires no low-temperature storage because it is stable even at ambient temperature over a long period of time.

A fluid food prepared by dissolving one bag of the product in about 150-300 ml of water can be administered through nasal cavity, esophagus, stomach or intestine by means of intubation feeding.

### Example 15

### Tablet

Fifty parts by weight of salicyclic acid was homogeneously mixed with a powder obtained by the method in Example 4, and 8 parts by weight of corn starch, and the mixture was tabletted with a tablet machine to obtain a tablet with 5.25 mm in thickness and 680 mg in weight.

The tablet had a relatively low hygroscopicity, a sufficient physical strength, and an excellent degradability in water.

As evident from the above, the present invention elucidates a novel maltotetraose-forming amylase, as well as establishing its preparation and uses.

The maltotetraose-forming amylase has the features that it has a relatively wide optimum- and stable-pH; that it has a relatively high optimum- and stable-temperature; and that it can be produced in large amount by Pseudomonas stutzeri MO-19. With these features, the amylase can be favorably used in the preparation of high maltotetraose and high maltotetraitol from amylaceous substances. Thus, the maltotetraose-forming amylase is significant in industrial uses.

Furthermore, high maltotetraose and high maltotetraitol obtained by hydrogenating the former are characterized in that they can be used in food products as a sweetener with a moderate sweetness, as well as a body-imparting agent, viscosity-controlling agent, humectant, gloss-imparting agent and nutrient supplement.

## Claims

1. Maltotetraose-forming amylase having the following physicochemical properties:
(a) Action
Acting on starch to produce primarily maltotetraose
(b) Substrate specificity
Acting on starch, amylose, amylopectin, glycogen and β-limit dextrin; but substantially not acting on cyclodextrin, dextran, pullulan and elsinan
(c) Optimum pH
About pH 7.0-7.5 at 40°C for 20 minutes
(d) Stable pH
About pH 6.5-9.5 at 40°C for 1 hour
(e) Optimum temperature
About 50°C at pH 7.0 for 20 minutes
(f) Stable temperature
Up to about 45°C at pH 7.0 for 1 hour. The activity is substantially retained at about 50°C in the presence of Ca²⁺, while at about 55°C about 70% of the activity is retained.
(g) Inhibition and stabilization
Inhibited by Hg⁺
Thermally stabilized by Ca²⁺
(h) Molecular weight
46,000±1,000 on SDS-polyacrylamide gel electrophoresis
47,000±2,000 on ultracentrifugal analysis
(i) Isoelectric point (pI)
About pI 4.8 on polyacrylamide gel isoelectric electrophoresis
(j) Ultraviolet absorption
About 275-280 nm.

2. A maltotetraose-forming amylase as claimed in claim 1, which has a crystalline form as shown in FIG. 2.

3. A maltotetraose-forming amylase as claimed in claim 1, wherein the amino acid sequence of the N-terminal of said maltotetraose-forming amylase is aspartic acid-glutamine-alanine-glycine-lysine-serine-proline-asparagine-alanine-valine.

4. A process for preparing a maltotetraose-forming amylase as defined in claim 1, which process comprises:
culturing a bacterium of the genus Pseudomonas capable of producing said maltotetraose-forming amylase with a nutrient culture medium to produce the amylase; and
recovering the desired amylase.

5. A process as claimed in claim 4, wherein said bacterium is of the species Pseudomonas stutzeri.

6. A process as claimed in claim 5, wherein said bacterium is Pseudomonas stutzeri MO-19 (FERM BP-1682).

7. A process as claimed in claim 4, wherein the amino acid sequence of the N-terminal of said maltotetraose-forming amylase is aspartic acid-glutamine-alanine-glycine-lysine-serine-proline-asparagine-alanine-valine.

8. A process for preparing a product having a high-maltotetraose content, including the step of subjecting an amylaceous substance to the action of a maltotetraose-forming amylase as claimed in any one of claims 1 to 3.

9. A process as claimed in claim 8, wherein said maltotetraose-forming amylase is used together with a starch-degrading enzyme.

10. A process for preparing a product having a high-maltotetraitol content, which process comprises:
subjecting an amylaceous substance to the action of a maltotetraose-forming amylase as claimed in claim 1, 2 or 3, to produce a product having a high maltotetraose content; and
hydrogenating the resultant product.

11. A process for preparing a food product, including the step of adding to the food product either a product having a high-maltotetraose content and obtained by a process as claimed in claims 8 and 9, or a product having a high-maltotetraitol content obtained by a process as claimed in claim 10.

## Patentansprüche

1. Maltotetraosebildende Amylase mit den folgenden physikochemischen Eigenschaften:
a) Wirkung
Wirkung auf Stärke um hauptsächlich Maltotetraose zu produzieren
b) Substratspezifität
Wirkung auf Stärke, Amylose, Amylopektin, Glycogen und β-begrenztes Dextrin, jedoch im wesentlichen keine Wirkung auf Cyclodextrin, Dextran, Pullulan und Elsinan
c) pH-Optimum
Etwa pH 7,0 - 7,5 bei 40°C 20 Minuten lang
d) Stabiler pH
Etwa pH 6,5 - 9,5 bei 40°C 1 Stunde lang
e) Temperaturoptimum
Etwa 50°C bei pH 7,0 20 Minuten lang
f) Stabile Temperatur
Bis zu etwa 45°C bei pH 7,0 1 Stunde lang. Die Aktivität wird im wesentlichen bei etwa 50°C in Gegenwart von Ca²⁺ beibehalten, während bei etwa 55°C etwa 70% der Aktivität beibehalten wird.
g) Inhibierung und Stabilisierung
Durch Hg⁺ inhibiert
Durch Ca²⁺ thermisch stabilisiert
h) Molekulargewicht
46.000 ± 1.000 bei SDS-Polyacrylamidgelelektrophorese
47.000 ± 2.000 bei Ultrazentifugenanalyse
i) Isoelektrischer Punkt (pI)
Etwa pI 4,8 bei isoelektrischer Elektrophorese mit Polyacrylamidgel
j) Ultraviolette Absorption
Etwa 275 - 280nm.

2. Maltotetraosebildende Amylase nach Anspruch 1, die eine kristalline Form, wie in Figur 2 dargestellt, besitzt.

3. Maltotetraosebildende Amylase nach Anspruch 1, bei der die Aminosäuresequenz des N-Endes der maltotetraosebildenden Amylase Asparaginsäure-Glutamin-Alanin-Glycin-Lysin-Serin-Prolin-Asparagin-Alanin-Valin ist.

4. Verfahren zur Herstellung einer, wie in Anspruch 1 definierten, maltotetraosebildenden Amylase, wobei das Verfahren die folgenden Schritte umfaßt:
Kultivierung eines Bakteriums der Gattung Pseudomonas, das in der Lage ist, die maltotetraosebildende Amylase zu produzieren mit einem Nährkulturmedium um die Amylase zu produzieren, und Gewinnung der gewünschten Amylase.

5. Verfahren nach Anspruch 4, bei dem das Bakterium der Spezies Pseudomonas stutzeri angehört.

6. Verfahren nach Anspruch 5, bei dem das Bakterium Pseudomonas stutzeri MO-19 (FERM BP-1682) ist.

7. Verfahren nach Anspruch 4, bei dem die Aminosäuresequenz des N-Endes der maltotetraosebildenden Amylase AsparaginsäureGlutamin-Alanin-Glycin-Lysin-Serin-Prolin-Asparagin-Alanin-Valin ist.

8. Verfahren zur Herstellung eines Produkts mit einem hohen Maltotetraosegehalt, das den Schritt einschließt bei dem eine stärkeartige Substanz der Wirkung einer maltotetraosebildenden Amylase, wie sie in einem der Ansprüche 1 bis 3 beansprucht wird, unterworfen wird.

9. Verfahren nach Anspruch 8, bei dem maltotetraosebildende Amylase zusammen mit einem stärkeabbauenden Enzym eingesetzt wird.

10. Verfahren zur Herstellung eines Produkts mit einem hohen Maltotetraitolgehalt, wobei das Verfahren die folgenden Schritte einschließt:
Unterwerfung einer stärkeartigen Substanz der Wirkung einer maltotetraosebildenden Amylase, wie in Anspruch 1, 2 oder 3 beansprucht, um ein Produkt mit einem hohen Maltotetraosegehalt herzustellen, und
Hydrierung des resultierenden Produkts.

11. Verfahren zur Herstellung eines Lebensmittels, das den Schritt einschließt das Lebensmittel entweder mit einem Produkt mit einem hohen Maltotetraosegehalt und das durch ein Verfahren wie in den Ansprüchen 8 und 9 beansprucht, erhalten wurde, oder mit einem Produkt mit einem hohen Maltotetraitolgehalt, das durch ein Verfahren erhalten wurde, wie in Anspruch 10 beansprucht, zu versetzen.

## Revendications

1. Amylase formant du maltose et ayant les propriétés physicochimiques suivantes :
(a) Action
Agissant sur l'amidon en produisant principalement du maltotétraose.
(b) Spécificité vis à vis du substrat
Agissant sur l'amidon, l'amylose, l'amylopectine, le glycogène et la dextrine-limite β ; mais n'agissant pratiquement pas sur la cyclodextrine, le dextrane, le pullulane et l'elsinane
(c) pH optimum
pH d'environ 7,0 à 7,5 à 40°C pendant 20 minutes.
(d) pH stable
pH d'environ 6,5 à 9,5 à 40°C pendant 1 heure.
(e) Température optimum
Environ 50°C à pH 7,0 pendant 20 minutes.
(f) Température stable
Jusqu'à environ 45°C à pH 7,0 pendant 1 heure. L'activité est pratiquement maintenue à environ 50°C en présence de Ca²⁺, tandis qu'à environ 55°C, environ 70 % de l'activité est maintenue.
(g) Inhibition et stabilisation
Inhibé par Hg⁺
Stabilisé du point de vue thermique par Ca²⁺.
(h) Poids moléculaire
46 000 ± 1000 selon l'électrophorèse sur gel de polyacrylamide en présence de SDS.
47 000 ± 2000 selon l'analyse par ultracentrifugation.
(i) Point isoélectrique (pI)
pI d'environ 4,8 par isoélectrofocalisation sur gel de polyacrylamide.
(j) Absorption en ultraviolet
Environ 275 à 280 nm.

2. Amylase formant du maltotétraose comme revendiqué dans la revendication 1, ayant une forme cristalline telle que celle présentée sur la FIG. 2.

3. Amylase formant du maltotétraose comme revendiqué dans la revendication 1, dans laquelle la séquence d'acides aminés N-terminale de la dite amylase formant du maltotétraose est acide aspartique-glutamine-alanine-glycine-lysine-sérine-proline-asparagine-alanine-valine.

4. Procédé de préparation de l'amylase formant du maltotétraose comme défini dans la revendication 1, ce procédé comprenant :
la culture d'une bactérie du genre Pseudomonas capable de produire la dite amylase formant du maltotétraose à l'aide d'un milieu de culture nutritif pour produire l'amylase ; et
la récupération de l'amylase souhaitée.

5. Procédé comme revendiqué dans la revendication 4, dans lequel la dite bactérie est du genre Pseudomonas stutzeri.

6. Procédé comme revendiqué dans la revendication 5, dans lequel la dite bactérie est du genre Pseudomonas stutzeri MO-19 (FERM BP-1682).

7. Procédé comme revendiqué dans la revendication 6, dans lequel la séquence d'acides aminés N-terminale de la dite amylase formant du maltotétraose est acide aspartique-glutamine-alanine-glycine-lysine-sérine-proline-asparagine-alanine-valine.

8. Procédé de préparation d'un produit ayant un contenu en maltotétraose élevé, comprenant une étape au cours de laquelle une substance amylacée est soumise à l'action de l'amylase formant du maltotétraose telle que revendiquée dans l'une quelconque des revendications 1 à 3.

9. Procédé comme revendiqué dans la revendication 8, dans lequel la dite amylase formant du maltotétraose est utilisée en même temps qu'une enzyme dégradant l'amidon.

10. Procédé de préparation d'un produit ayant un contenu en maltotétraitol élevé, ce procédé comprenant :
la soumission d'une substance amylacée à l'action de l'amylase formant du maltotétraose comme revendiqué dans les revendications 1, 2 ou 3 pour produire un produit ayant un contenu élevé en maltotétraose ; et
l'hydrogénation du produit obtenu.

11. Procédé de préparation d'un produit alimentaire, comprenant une étape au cours de laquelle un produit ayant un contenu en maltotétraose élevé et obtenu par un procédé comme revendiqué dans les revendications 8 et 9, ou un produit ayant un contenu en maltotétraitol élevé et obtenu par un procédé comme revendiqué dans la revendication 10, est ajouté au produit alimentaire.
